# EUROPEAN PATENT SPECIFICATION

(11) **EP 3 853 360 B1**
(45) Date of publication and mention of the grant of the patent: **15.07.2026**
(21) Application number: 19801323.7
(22) Date of filing: 08.11.2019
(51) Int. Cl.: C12N 9/76, C12N 15/10

(54) **TRYPSIN MUTANT FOR C- AND N-TERMINAL MODIFICATION OF POLYPEPTIDES**
TRYPSIN-MUTANTE ZUR C- UND N-TERMINALEN MODIFIZIERUNG VON POLYPEPTIDEN
MUTANT DE TRYPSINE POUR LA MODIFICATION DE POLYPEPTIDES PAR LES TERMINAUX C ET N

(30) Priority: 08.11.2018 EP 18205212
(43) Date of publication of application: 28.07.2021
(73) Proprietor: BioPharma Translationsinstitut Dessau Forschungs GmbH, 06861 Dessau-Rosslau (DE)
(72) Inventor: BORDUSA, Frank, 06242 Rossbach (DE); LIEBSCHER, Sandra, 06120 Halle (Saale) (DE); MEYER, Christoph, 01454 Radeberg (DE)
(74) Representative: Isarpatent
(86) International application number: PCT/EP2019/080673
(87) International publication number: WO 2020/094840

(56) References cited:
- WO-A1-2006/015879
- WO-A1-2007/031187
- WO-A1-2011/098400
- CHARLES S. CRAIK ET AL: "Redesigning Trypsin: Alteration of Substrate Specificity", SCIENCE, vol. 228, 19 April 1985 (1985-04-19), pages 291 - 298, XP055577006
- W. SCOTT WILLETT ET AL: "Engineered Metal Regulation of Trypsin Specificity", BIOCHEMISTRY, vol. 34, no. 7, 1 February 1995 (1995-02-01), pages 2172 - 2180, XP055576987, ISSN: 0006-2960, DOI: 10.1021/bi00007a010
- XU S ET AL: "Enzymatic coupling of specific peptides at nonspecific ligation sites: effect of Asp189Glu mutation in trypsin on substrate mimetic-mediated reactions", JOURNAL OF ORGANIC CHEMISTRY,, vol. 66, 9 March 2001 (2001-03-09), pages 1627 - 1632, XP002218036, ISSN: 0022-3263, DOI: 10.1021/JO001243C
- KURTH T ET AL: "Engineering the S1' subsite of trypsin: design of a protease which cleaves between dibasic residues", BIOCHEMISTRY, AMERICAN CHEMICAL SOCIETY, vol. 37, 18 August 1998 (1998-08-18), pages 11434 - 11440, XP002218040, ISSN: 0006-2960, DOI: 10.1021/BI980842Z

## Description

### Field of the Invention

The present invention relates to a mutated trypsin comprising an amino acid substitution both at position K60 and D189, and amino acid substitution at position Y39 and Y59, having a preferred enzyme recognition site TYR-ARG-Xaa-HIS, wherein Xaa is any amino acid. The present invention further relates to a nucleotide sequence coding for the mutated trypsin of the present invention, to a method for preparing C-terminal transacylated target polypeptides by using the mutated trypsin of the present invention, and to a method for preparing a N-terminal transacylated target polypeptide by using the mutated trypsin of the present invention.

### Background of the Invention

There is a high demand to provide polypeptides with covalent modifications and methods to introduce specific covalent modifications to polypeptides.

In addition to the various purely chemical methods, which are generally non-regiospecific or lead to global modifications of the polypeptide, there are also molecular biological and enzymatic methods, or combinations of chemical and enzymatic methods for the site-specific modification of polypeptides.

Orthogonal modification methods result in chemoselectively labeled or modified polypeptides. Orthogonality for position-specific modification of polypeptides can only occur in exceptional cases without prior manipulation of the corresponding polypeptide at the genetic level. For example, recognition sequences for subsequent enzyme-catalyzed modification are integrated at the genetic level into the polypeptide sequence, which can be used for position-specific modification (the position of the introduced label is determined by the position of the recognition sequence).

Enzymatic methods for modifying polypeptides use intrinsic properties of enzymes such as the recognition of certain amino acid sequences or functionalities after the introduction of the corresponding recognition sequences by means of site-directed mutagenesis. Regiospecificity is generated by the high substrate specificity of the respective enzymes. The fact that each polypeptide modified in this way has only one recognition sequence gives the method a regio- and chemoselective character, since usually only one amino acid is modified within a consensus sequence.

Proteases can be useful enzymes for the modification of polypeptides. Trypsin is a serine protease which specifically cleaves carboxy-terminal of basic amino acid residues. The active center consists of Ser195, Asp102 and His57 (catalytic triad). Ser195 forms an acyl enzyme intermediate with the substrate to be cleaved and is thus significantly involved in the protease reactivity. This acyl enzyme intermediate can be attacked by variable nucleophiles such as water (peptide hydrolysis), amines (peptide aminolysis), alcohols and thiols (peptide (thio)esterification). By forming a covalent acyl enzyme intermediate, the serine protease trypsin thus meets all the requirements for kinetically controlled acyl transfer.

In contrast to peptide cleavage, peptide bond linkage is a two-substrate reaction. The acyl donor binds at the S-binding site of the enzyme, while the acyl acceptor interacts with the S' binding region.

The kinetically controlled C-terminal modification of polypeptides via stable amide bonds is based on transamidation. The C-terminal end region of the polypeptide to be labeled forms the acyl enzyme intermediate with the trypsin variant, which can then be attacked nucleophilically by the labeled acyl acceptor.

In WO 2006/015879 A1 a trypsin mutant K60E/D189K/N143H/E151H is described which recognizes histidine side chains zinc-ion induced in the P₂'-position of a peptide with the restriction site -Tyr-Arg-His- hydrolyses specifically the recognition sequence -YRH- between amino acids tyrosine und arginine in the presence of zinc-ions.

In view of the known methods for C-terminal modification of polypeptides there is a further need for methods for the C-terminal modification of polypeptides. In particular there is a need to provide a mutated trypsin which recognizes histidine functionalities in the P₃' position of the substrate.

### Summary of the Invention

The inventors of the present invention have found that it is possible to use special trypsin mutants for the specific C-terminal modification of polypeptides.

Thus, a first aspect of the present invention provides a mutated trypsin comprising an amino acid substitution both at position K60 and D189, and amino acid substitution at position Y39 and Y59. According to another aspect, the present invention relates to the use of a polypeptide comprising a target polypeptide and a restriction site peptide comprising the regognition site TYR-ARG-Xaa-HIS, wherein Xaa is any amino acid, wherein said restriction site peptide overlaps with the target polypeptide by the amino acid TYR at the C-terminal end of said target polypeptide as a substrate of a mutated trypsin according to the first aspect of the present invention.

According to a further aspect, the present invention provides a nucleotide sequence coding for the mutated trypsin of the present invention.

According to a further aspect, the present invention provides a method for preparing a C-terminal transacylated target polypeptide comprising the steps of:
(a) providing a polypeptide comprising a target polypeptide and a restriction site peptide comprising the recognition site TYR-ARG-Xaa-HIS, wherein Xaa is any amino acid, and wherein said restriction site peptide overlaps with the target polypeptide by the amino acid TYR at the C-terminal end of said target polypeptide,
(b) bringing said peptide into contact with a trypsin mutant of the present invention under conditions allowing for endoproteolytic cleavage after TYR and formation of an endoprotease target polypeptide peptide-acyl-intermediate,
(c) adding an appropriate nucleophile and
(d) upon nucleophilic attack and binding of said nucleophile to the C- terminus of the target polypeptide releasing the mutated trypsin from the endoprotease target polypeptide-acyl-intermediate.

Preferably, said nucleophile is selected from the group consisting of primary amines, imines, secondary amines, thiol and hydroxyl.

More preferably said nucleophile comprising modification is selected from the group consisting of an amino acid amide, a peptide, a peptide amide, a label, a labeled amino acid amide, a labeled peptide, a labeled peptide amide, and polyethylene glycole.

Even more preferably the nucleophile is a click-anchor comprising at least one additional functional group A, wherein the at least functional group A is capable under appropriate conditions to form at least one covalent bond to a complementary functional group B. Preferably, the at least one additional functional group A of the click-anchor is selected from the group consisting of an aldehyde, an azide, a C-C double bond, a C-C triple bond, a C-N triple bond, a diene, an imine, a ketone, a tetrazine, a thioketone and a thiole.

Preferably, the functional group B is selected from the group consisting of an aminooxy, an azide, a C-C double bond, a C-C triple bond, a O-Chinonmethid, a C-N triple bond, a cystamine, a diene, a hydrazide, an iodophenyl, a nitrilimine, a nitrilioxide, a phosphine, a phosphite, a tetrazine, a thiocarboxylic acid and a thiole.

According to a further aspect, the present invention provides a method for preparing a N-terminal transacylated target polypeptide comprising the steps of:
(a) providing a polypeptide comprising a target polypeptide and a tripeptide ARG-Xaa-HIS N-terminally fused to the target polypeptide, wherein Xaa is any amino acid,
(b) providing a reactive acyl donor substrate,
(c) bringing the reactive acyl donor substrate into contact with the mutated trypsin according to the present invention under conditions allowing for acylation of Ser195 of the mutated trypsin by the reactive acyl donor substrate,
(d) adding the polypeptide from step (a),
(e) upon nucleophilic attack and binding of said polypeptide, releasing the mutated trypsin and forming a N-terminally transacylated target polypeptide.

Further preferred and exemplary embodiments of the present invention are indicated in the dependent claims and the following detailed description, which, however do not restrict the scope of the invention and only help to understand and explain the features of the present invention. Deviations and modifications on these particular features, particular with regard to other aspects of the invention, can be made without departing from the cops of the invention.

### Description of the Figures

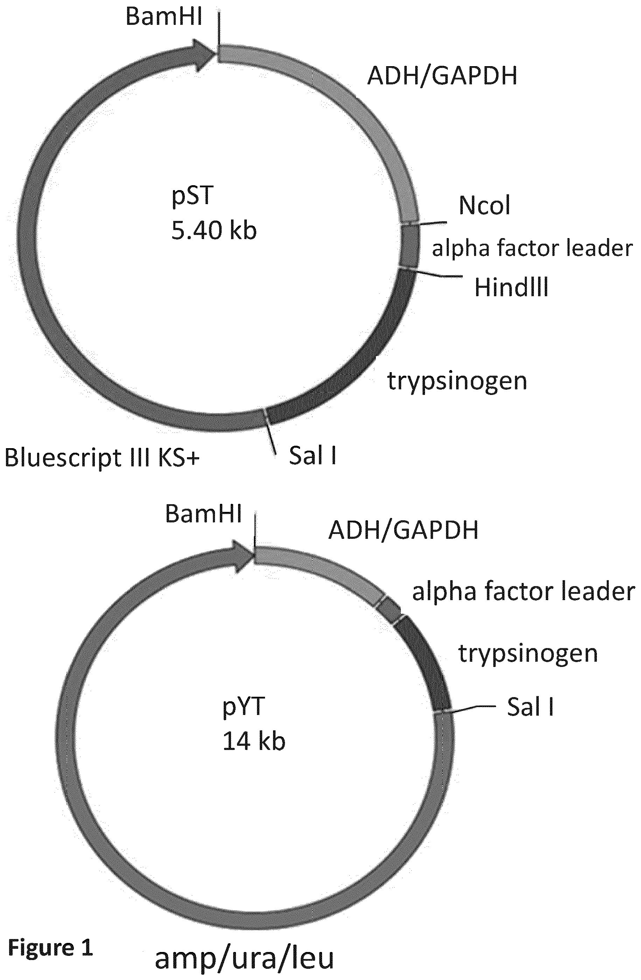

Figure 1: Plasmid used for the site-directed mutagenesis of trypsin.

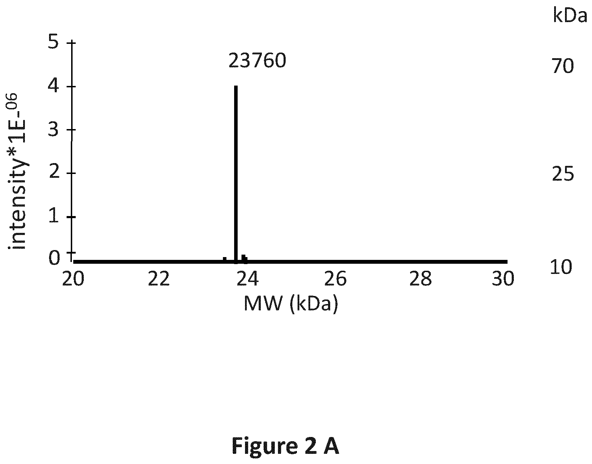

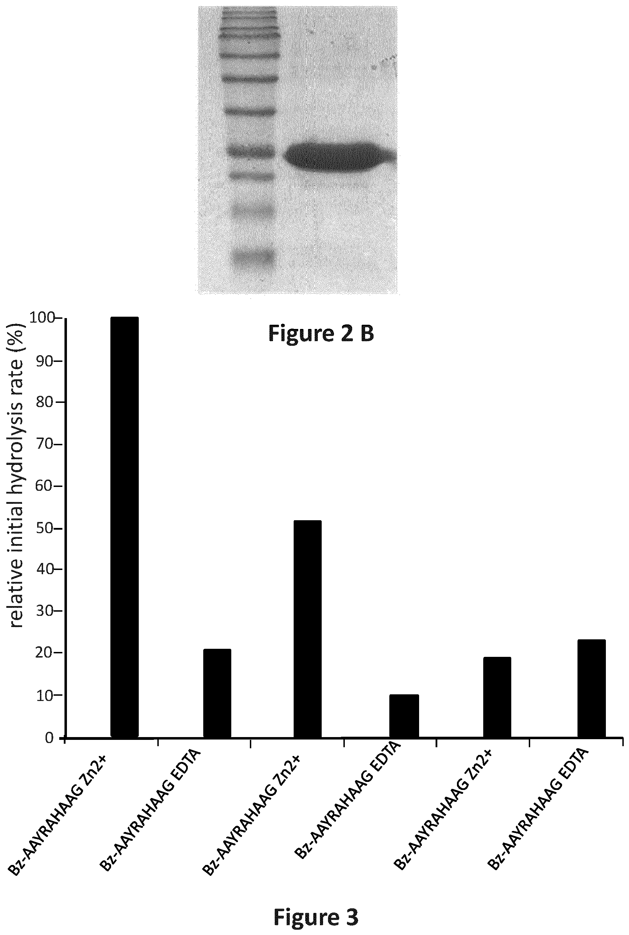

Figure 2: Chromatographic purification of activated trypsin mutant, its mass spectrometric and gel electrophoretic identification. A) Mass spectrometric analysis of trypsin mutant MW_{cal}.: 23,759 Da MW_{det}: 23,760 Da [M+H⁺]. B) SDS-PAGE from Tn K60E/D189K/Y39H/Y59H.
Figure 3: Specificity tests with the peptides Bz-AAYRAHAAG, Bz-AAYRHAAG, Bz-AAYRAAG as a function of zinc ions or in the presence of EDTA. The positions S₂' and S₃' were varied. The measurements were carried out either in the presence of zinc ions or in the presence of EDTA. Y39H/Y59H/K60E/D180K was used as trypsin variant. 1 mM peptide, 10 µM trypsin variant, 0.1 M HEPES, 0.1 M NaCl, 0.01 M CaCl₂, 100 µM ZnCl₂/EDTA; pH 8. The hydrolysis rate of the peptide Bz-AAYRHAAG in the presence of ZnCl₂ was set to 100%.

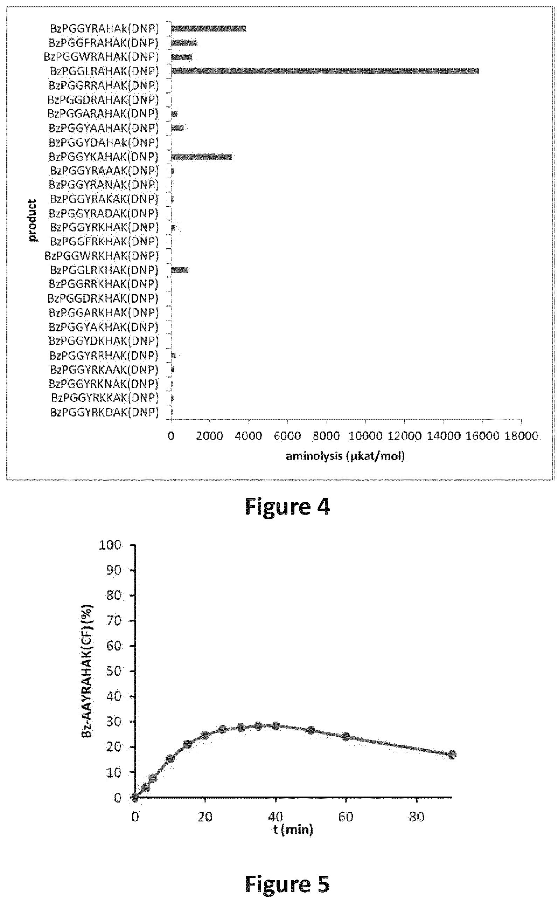

Figure 4: Specificity studies on the trypsin variant using the transamidation reaction. 2 µM trypsin variant, 100 µM acyl donor, 200 µM acyl acceptor, 100 µM ZnCl₂, buffer (100 mM HEPES, 100 mM NaCl, 10 mM CaCl₂, pH 7.8; e.g. BzPGGYRAHAK as acyl-donor was converted with RAHAK(DNP) as acyl acceptor using the said trypsin variant.
Figure 5: Enzymatically catalyzed C-terminal modification with the model peptides Bz-AAYRAHAAG (acyl donor,) and RAHAK(CF) (acyl acceptor). The product yield (in %, Y-axis) is plotted as a function of time (X-axis).

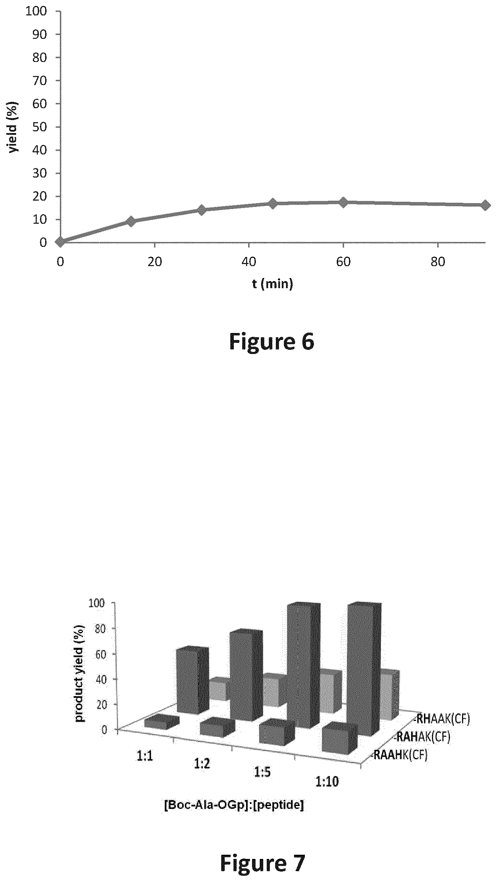

Figure 6: Enzymatically catalyzed transamidation between anti-Her2-Fab-YRAH and RAHAK(DNP). The yield of product (Fab-YRAHAK(DNP) against time (in min) is plotted, the maximum yield is 17%.
Figure 7: Trypsin mutant catalyzed acyl transfer of Boc-Ala-OGp and histidine-containing peptides using varying concentration ratios of the acyl acceptor. 0.1 M HEPES, 0.1 M NaCl, 0.01 M CaCl₂, 100 µM ZnCl₂; pH 7.8; 80 min; 30 °C. Boc-Ala-OGp: 100 µM; [Peptide]: 100 µM (1:1); 200 µM (1:2); 500 µM (1:5), 1 mM (1:10); [Trypsin mutant]: 10 µM.

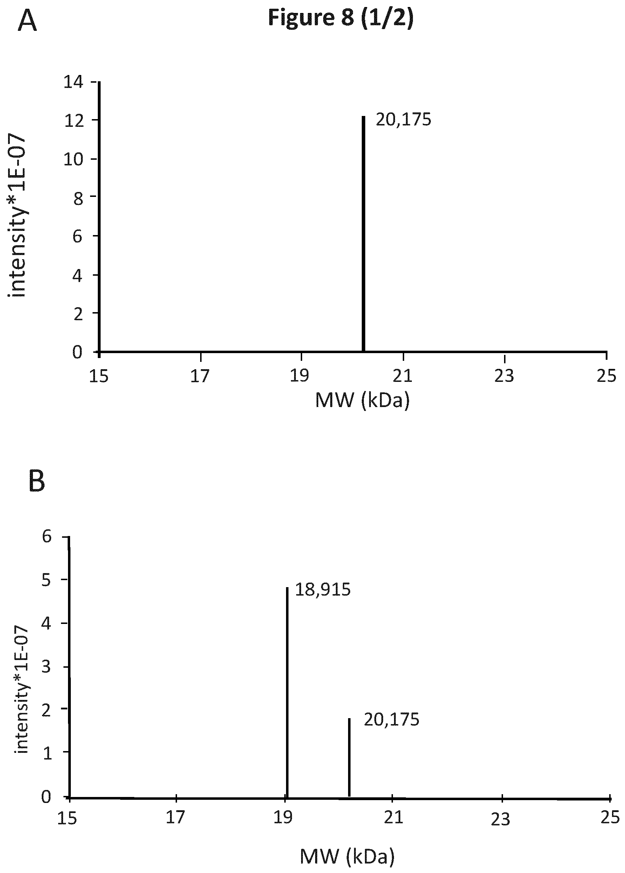

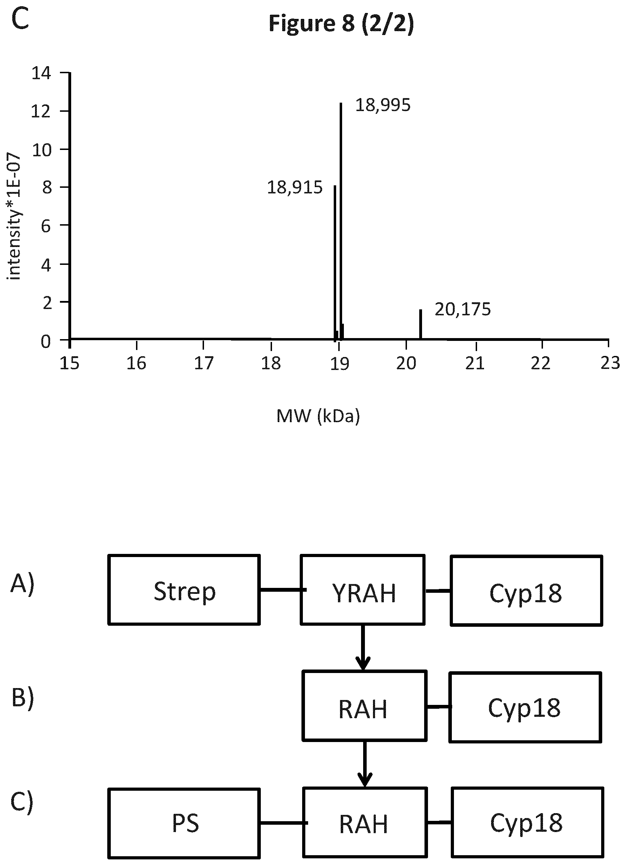

Figure 8: Mass spectrometric analysis of the modification of RAH-cyclophilin18-YRH catalyzed by trypsin mutant with 4-pentynoic acid (PS-OGp). 0.1 M HEPES; 0.1 M NaCl; 10 mM CaCl₂; 50 µM ZnCl₂; pH 7.8; 30°C. [YRAH-Cyp18-YRH]: 200 µM; [Trypsin mutant]: 10 µM; [PS-OGp]: 1 mM. A: Strep-YRAH-Cyp18-YRH MW_{cal}.: 20,174 Da; MW_{det.}: 20,175 Da [M+H⁺]. B: RAH-Cyp18-YRH MW_{cal}.: 18,914 Da; MW_{det.}: 18,915 Da [M+H⁺]. C: PS-YRAH-Cyp18-YRH. MW_{cal}.: 18,994 Da; MW_{det.:}. 18,995 Da [M+H⁺]. Additionally shown is a schematic representation of the reaction process of the modification A) educt; B) hydrolysis product; C) product of N-terminal modification.

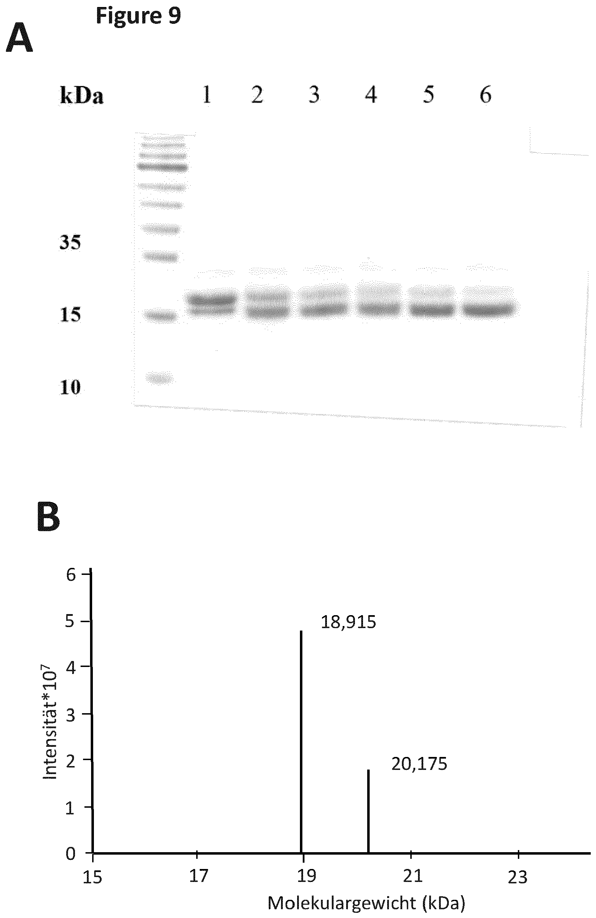

Figure 9: Tn K60E/D189K/Y39H/Y59H-catalyzed cleavage of the Strep fusion peptide from Strep-YRAH-cyclophilin18-YRHAAG. Reaction conditions: 0.1 M HEPES, 0.1 M NaCl, 0.01 M CaCl₂, 100 µM ZnCl₂; 10 µM Tn K60K/D189K/Y39H/Y59H; pH 8; 30 °C; 5 h. A: Polyacrylamide electrophoresis. 1: 0 h; 2: 1 h; 3: 2 h; 4: 3 h; 5: 4 h; 6: 5 h reaction time. B: Determination of molecular weight after 2h. RAH-cyclophilin18-YRHAAG: MW_{calc.}: 18,914 Da; MW_{det.}: 18,915 Da [M+H⁺]; Strep-YRAH-cyclophilin18-YRHAAG: MW_{calc.}: 20,174 Da; MW_{det.}: 20,175 Da [M+H⁺].

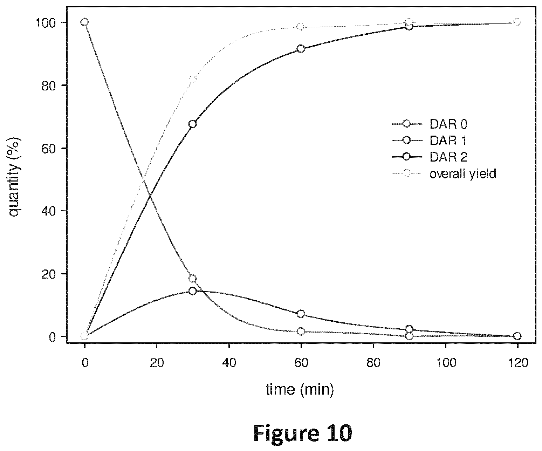

Figure 10: Reaction course of the trypsin mutant catalyzed N-terminal modification of full length anti-Her2-antibody RAH-Trastuzumab with Dan-Ahx-OGp. DAR 0, DAR 1 and DAR 2 define the Drug-Antibody-Ratio. Dar 0: starting anti-Her2-antibody (educt); DAR 1: single modified anti-Her2-antibody (one Dan-Ahx moiety at one N-terminus of the antibody); DAR 2: full modified anti-Her2-antibody (one Dan-Ahx moiety at both N-termini of the antibody). Reaction conditions: 0.1 M HEPES, 0.1 M NaCl, 0.01 M CaCl₂, 100 µM ZnCl₂; pH 7.8; 30 °C. [Dan-Ahx-OGp]: 1 mM; [RAH-Trastuzumab]: 32 µM; [Trypsin mutant]: 30 µM. Analysis conditions: HIC (Hydrophobic Interaction Chromatography): A 1.2 M (NH₄)₂SO₄ 25 mM NaPᵢ (pH 5.0) B 25 mM NaPᵢ (pH 5.0) 100% A => 100% B in 15 Minuten, λ=280 nm.

### Detailed Description

To facilitate an understanding of the invention, a brief discussion of the terminology used in connection with the invention will be provided. The present disclosure uses the terminology of Schechter, J., and Berger, A., Biochem. Biophys. Res. Commun. 27 (1967) 157-162, to describe the location of various amino acid residues on the peptide substrate and individual binding sites within the active site of a corresponding proteolytic enzyme.

According to the terminology proposed by Schechter, J. and Berger, A., supra, the amino acid residues of the peptide substrate are designated by the letter "P". The amino acids of the substrate on the N-terminal side of the peptide bond to be cleaved (the "cleavage site" or "recognition site") are designated Pₙ...P₃, P₂, P₁ with Pₙ being the amino acid residue furthest from the cleavage site. Amino acid residues of the peptide substrate on the C-terminal side of cleavage site are designated P₁', P₂', P₃',...Pₙ' with Pₙ' being the amino acid residue furthest from the cleavage site. Hence, the bond which is to be cleaved (the "cleavage site" or "recognition site") is the P₁-P₁' bond.

The generic formula for the amino acids of the substrate of an endopeptidase (like for example trypsin) is as follows:
Pₙ-P₃-P₂-P₁-P₁'-P₂'-P₃'Pₙ'

The designation of the substrate binding sites of an endopeptidase is analogous to the designation of amino acid residues of the peptide substrate. However, the binding sub-sites of an endopeptidase are designated by the letter "S" and can include more than one amino acid residue. The substrate binding sites for the amino acids on the N-terminal site of the cleavage site are labeled Sₙ..., S₃, S₂, S₁. The substrate binding sub site for the amino acids on the carboxy side of the cleavage site are designated S₁', S₂', S₃',...Sₙ'. Hence, in an endopeptidase, the S₁' sub site interacts with the P₁' group of the peptide substrate and the incoming nucleophile.

A generic formula for describing substrate binding sites of an endopeptidase is:
Sₙ-S₃-S₂-S₁-S₁'-S_{2'}-S₃'-Sₙ'

The S₁ binding site binds the side chain of the penultimate amino acid, P₁, of the peptide substrate, in case of a trypsin mutant according to this invention the amino acid TRY. The S₁' binding site interacts with the side chain of P₁', in the present case with ARG. Likewise, the S₂' binding site interacts with the side chain of the Xaa residue in position P₂'.

The term "variant" refers to polypeptides having amino acid sequences that differ to some extent from a native polypeptide sequence. Ordinarily, a variant amino acid sequence will possess at least about 80% homology with the corresponding parent trypsin sequence, and preferably, it will be at least about 90%, more preferably at least about 95% homologous with such corresponding parent trypsin sequence. The amino acid sequence variants possess substitutions, deletions, and/or insertions at certain positions within the amino acid sequence of the native amino acid sequence. Preferably sequence homology will be at least 96% or 97%.

"Homology" is defined as the percentage of residues in the amino acid sequence variant that are identical after aligning the sequences and introducing gaps, if necessary, to achieve the maximum percent homology. Methods and computer programs for the alignment are well known in the art. One such computer program is "Align 2," authored by Genentech, Inc., which was filed with user documentation in the United States Copyright Office, Washington, DC 20559, on December 10, 1991.

Preferably, a variant of trypsin as disclosed in the present invention in comparison to the corresponding wild-type sequence, comprises 20 amino acid substitutions or less, more preferred 15 amino acid substitutions or less, also preferred 10 amino acid substitutions or less, and also preferred 6 amino acid substitutions or less. These substitutions preferably are outside and in addition to the substitution positions identified below (positions K60, D189, Y39 or Y59). The wild-type sequence of trypsin may be derived from Rattus norvegicus or other rat species, but also from other origin such as well-known bovine or porcine trypsin wild-type sequences.

### Mutated trypsin

A first aspect of the present invention provides a mutated trypsin comprising an amino acid substitution both at position K60 and D189, and amino acid substitution at position Y39 and Y59 according to the chymotrypsin nomenclature which corresponds to positions 43, 171, 22, and 42, respectively, of the trypsin sequence given in SEQ ID NO:1.

The term "mutated trypsin" as used herein also comprises variants of the above defined mutated trypsin (which is based on the wild-type trypsin sequence of SEQ ID NO:1). Those variants of mutated trypsin can be based on other wild-type sequences as mentioned above, and may comprise amino acid substitutions of 20 amino acid substitutions or less, more preferred 15 amino acid substitutions or less, also preferred 10 amino acid substitutions or less, and also preferred 6 amino acid substitutions or less related to SEQ ID NO: 1 or other wild-type sequences. These substitutions preferably are outside and in addition to the substitution positions identified below (position K60, D189, Y39 and Y59).

In case that other wild-type sequences than that of SEQ ID NO: 1 are used as the basis for the mutated trypsin, it may happen that the positions of the amino acid substitution (K60, D189, Y39 and Y59) are shifted to another position. The skilled artisan, however, will know how to identify these altered positions and how to perform the substitutions as defined above in these wild-type sequences in order to arrive at a mutated trypsin according to the invention.

The skilled artisan further is familiar with the so-called chymotrypsin nomenclature as, e.g., described in Hartley, B.S., and Shotton, D.M., The Enzymes, P.D. Boyer (ed.), Vol. 3, (1971), pp. 323-373 and will have no problem in aligning the positions of a variant trypsin with positions given according to the chymotrypsin nomenclature to the corresponding ones of the trypsin sequence of SEQ ID No: 1.

Position 39 according to chymotrypsin nomenclature corresponds to position 22 of the sequence of mature anionic rat trypsin II from Rattus norvegicus as given in SEQ ID NO: 1.

Position 59 according to chymotrypsin nomenclature corresponds to position 42 of the sequence of mature anionic rat trypsin II from Rattus norvegicus as given in SEQ ID NO: 1.

Position 60 according to chymotrypsin nomenclature corresponds to position 43 of the sequence of mature anionic rat trypsin II from Rattus norvegicus as given in SEQ ID NO: 1.

Position 189 according to chymotrypsin nomenclature corresponds to position 171 of the sequence of mature anionic rat trypsin II from Rattus norvegicus as given in SEQ ID NO: 1.

Since the skilled artisan is used to express positions referring to the chymotrypsin nomenclature, therefore, in the following the references to specific sequence position, e.g., position K60 or simply position 60 are exclusively based on positions according to the chymotrypsin nomenclature.

The mutated trypsin according to the present invention is a trypsin comprising amino acid substitutions at both the positions K60 and D189 and amino acid substitutions at both the positions Y39 and Y59.

Preferably in the embodiments described herein the substitution in position K60 is by glutamic acid (Glu, E) or aspartic acid (Asp, D), most preferably the substitution is by glutamic acid (Glu, E).

Preferably in the embodiments described herein the substitution in position D189 is preferably by lysine (Lys, K), histidine (His, H), or arginine (Arg, R), most preferably the substitution is by lysine (Lys, K).

Preferably in the embodiments described herein the substitution in position Y39 is preferably by lysine (Lys, K), histidine (His, H), aspartic acid (Asp, D), glutamic acid (Glu, D) or arginine (Arg, R), most preferably the substitution is by histidine (His, H).

Preferably in the embodiments described herein, the substitution in position Y59 is preferably by lysine (Lys, K), histidine (His, H), aspartic acid (Asp, D), glutamic acid (Glu, D) or arginine (Arg, R), most preferably the substitution is by histidine (His, H).

Thus, preferably the mutated trypsin according to the present invention comprises either the amino acid E or the amino acid D in position 60, thus replacing the amino acid K normally present in position 60. Most preferably the amino acid K normally present in position 60 is replaced by E. It is also preferred that the mutated trypsin according to the present invention comprises either the amino acid K, the amino acid H, or the amino acid R in position 189, thus replacing amino acid D normally present in that position. Most preferably the amino acid D normally present in position 189 is replaced by K.

In a further preferred embodiment the mutated trypsin according to the present invention comprises mutations in positions 39, 59, 60, and 189. Preferred substitutions in this mutated enzyme are Y39H, Y59H, K60E or D, and D189K, H, or R.

In the most preferred embodiment the mutated trypsin according to the present invention comprises the following substitutions: Y39H, Y59H, K60E, and D189K.

The above described mutants of trypsin have very interesting and important properties. The mutants preferably recognize a recognition or cleavage site consisting of four amino acids TYR-ARG-Xaa-HIS, wherein Xaa is any amino acid. This restriction site is cleaved by the above mutants after TYR. Thus, using the novel trypsin mutants only one amino acid, i.e. the C-terminal TYR, will remain within the modified target polypeptide. Hence, the trypsin mutants according the present invention recognize specifically histidine functionalities in the P₃' position of the substrate.

### Use of a polypeptide as a substrate of a mutated trypsin

The present invention also relates to the use of a synthetic polypeptide comprising a target polypeptide and a restriction site peptide comprising the recognition site TYR-ARG-Xaa-HIS, wherein Xaa is any amino acid, wherein said restriction site peptide overlaps with the target polypeptide by the amino acid TYR at the C-terminal end of said target polypeptide as a substrate of a trypsin mutant according to the present invention.

### Nucleotide sequence coding for the mutated trypsin

In a further embodiment the present invention relates to nucleotide sequences coding for the novel trypsin mutants, to vectors comprising such mutants and to transformed host cells comprising such vectors.

### Method for preparing C-terminal transacylated target polypeptide

Also provided is a method of producing a C-terminally transacylated target polypeptide comprising the steps of: (a) providing a polypeptide comprising a target polypeptide and a restriction site peptide comprising the recognition site TYR-ARG-Xaa-HIS, wherein Xaa is any amino acid, and wherein said restriction site peptide overlaps with the target polypeptide by the amino acid TYR at the C-terminal end of said target polypeptide, (b) bringing said peptide into contact with a trypsin mutant according to the present invention under conditions allowing for endoproteolytic cleavage after TYR and formation of an endoprotease target polypeptide peptide-acyl-intermediate, (c) adding an appropriate nucleophile and (d) upon nucleophilic attack and binding of said nucleophile to the C-terminus of the target polypeptide releasing the mutated trypsin from the endoprotease target polypeptide-acyl-intermediate.

The modified trypsin of the invention is capable of improved transacylation when compared to the corresponding native trypsin. As used herein, "transacylation" is a reaction in which a peptide fragment C-terminal to the trypsin recognition site is exchanged for a nucleophile. Transacylation reactions include transthiolation, transesterification and transamidation reactions. "Transamidation" occurs when an amide bond is formed between the nucleophile and the target polypeptide substrate. In a transamidation reaction, the nucleophile is not necessarily an amino acid. "Transpeptidation" as an important subgroup of transamidation occurs when the nucleophile is an amino acid, or amino acid derivative, such as an amino acid ester, or amino acid amide or peptide. A general transacylation reaction according to the present invention is shown below.

In the first step, the enzyme attacks the peptide bond between TYR and ARG, displacing the more C-terminal amino acids and forming a covalent (an acyl) bond between the P1 residue (TYR) of the target polypeptide and the enzyme. This intermediate is referred to as a target polypeptide "peptide-acyl-enzyme intermediate" or briefly as acyl-enzyme intermediate. In the presence of an appropriate nucleophile, under proper conditions, the enzyme causes the nucleophile to add to the cleaved peptide substrate to produce a transacylated product. It is believed that the nucleophile attaches to the carboxyl group of the acyl-enzyme intermediate and displaces the enzyme from the acyl-enzyme intermediate. In this manner, the nucleophile becomes linked to the carboxyl group of the peptide substrate.

Instead of undergoing a transacylation reaction, the acyl-enzyme intermediate might be deacylated by water to produce a hydrolysis product. The mutated trypsin of the present invention is designed to preferentially produce the transacylation product over the hydrolysis product.

The present invention also relates to a synthetic polypeptide comprising a target polypeptide and a restriction site peptide comprising the recognition site TYR-ARG-Xaa-HIS, wherein Xaa is any amino acid, wherein said restriction site peptide overlaps with the target polypeptide by the amino acid TYR at the C-terminal end of said target polypeptide. With other words it relates to a synthetic polypeptide comprising a target polypeptide and a restriction site peptide comprising the recognition site TYR-ARG-Xaa-HIS, wherein Xaa is any amino acid, wherein said restriction site peptide and said target polypeptide share the amino acid TYR at the C-terminal end of said target polypeptide.

The term target polypeptide thus refers to the peptide or polypeptide of the sequence Pₙ-P₃-P₂-TYR. The target polypeptide thus includes one amino acid of the recognition site for the mutated trypsin of this invention, i.e., the amino acid TYR. The term peptide does include polypeptides.

Synthetic peptide is a peptide with an artificial peptide sequence, e.g. a sequence designed by a scientist or by a computer. The present invention does not relate to naturally occurring polypeptides comprising the above-defined sequence motif TYR-ARG-Xaa-HIS. It merely relates, to synthetic, i.e. synthetically or recombinantly produced polypeptides which have been designed to comprise both a target polypeptide as well as a restriction site peptide to comprise the recognition site TYR-ARG-Xaa-HIS, wherein Xaa is any amino acid. The target polypeptide has the amino acid TYR as its C-terminal amino acid. The restriction site peptide comprises at least the amino acids ARG-Xaa-HIS, and optionally C-terminal thereto further amino acids. Thus the recognition site consisting of TYR-ARG-Xaa-HIS, overlaps with the target polypeptide by one amino acid (TYR) and by three amino acids (ARG-Xaa-HIS) with the restriction site peptide. As this skilled artisan will appreciate in principle any polypeptide comprising TYR-ARG-Xaa-HIS, wherein Xaa is any amino acid, may be used as a substrate for the trypsin mutants according to the present invention, e.g. in an effort of peptide ligation or in an effort of C-terminal peptide transacylation, e.g. for modification and/or labeling purposes.

Preferably a target polypeptide according to the present invention consists of 20 to 2000 amino acids. Also preferred is a target polypeptide consisting of 30 to 1500 amino acids. More preferred such target polypeptide consists of 40-1000 amino acids.

Preferred target polypeptides are polypeptides used in diagnostic or in therapeutic applications. Preferred target polypeptides for example comprise specific binding agents, like antibodies and fragments thereof. Also preferred are specific binding agents obtainable by phage display (see e.g., Allen, J.B., et al., TIBS 20 (1995) 511-516).

The term antibody refers to a polyclonal antibody, a monoclonal antibody, fragments of such antibodies, as well as to genetic constructs comprising the binding domain of an antibody. Any antibody fragment retaining essentially the same binding properties as the parent antibody can also be used.

Preferably the target polypeptide comprised in a recombinant polypeptide according to the present invention is a therapeutically active polypeptide.

Such therapeutically active polypeptide preferably is selected from the group consisting of a therapeutic antibody, erythropoietin and an interferon.

It is obvious to the skilled artisan that only such polypeptides will be used as target polypeptides which do not comprise the sequence motif TYR-ARG-Xaa-HIS naturally, wherein Xaa is any amino acid, as part of their sequence N-terminal to this desired recognition site. The skilled artisan will have no problems in excluding those polypeptides having a potential recognition site for a mutated trypsin of the present invention. In the alternative such potential internal recognition site sequence may be modified by routine mutation and/or cloning techniques to change and/or remove such un-desired internal recognition site.

In a preferred embodiment the polypeptide according to the present invention which comprises at or close to its C-terminus the sequence TYR-ARG-Xaa-HIS, wherein Xaa is any amino acid, is produced by recombinant methods. The skilled artisan will have no problem to engineer any desired target polypeptide which is accessible to recombinant production in a way to comprise at or close to the C-terminus the above defined restriction site of TYR-ARG-Xaa-HIS.

The restriction site peptide according to the present invention at least comprises the amino acids ARG-Xaa-HIS, wherein Xaa is any amino acid, at its N-terminus. It may contain additional amino acids C-terminal thereto which facilitate for example recombinant production or easy purification. In a further preferred embodiment the recombinant polypeptide will comprise as part of the restriction site peptide a so-called His-tag at its C-terminal end which allows for an easy purification by well-established chromatographic methods, e.g., use of hexa-His and Ni-NTA-chromatography (Hochuli, E., et al., J. Chromatogr. 411 (1987) 177-184).

Preferably the above described trypsin mutants are used in a method for C-terminal acylation of a peptide substrate. As the skilled artisan will appreciate, such peptide substrate will comprise a recognition site for a mutated trypsin according to this invention and the method will comprise the steps of providing an appropriate peptide substrate, bringing said peptide substrate into contact with a trypsin mutant according to the present invention under conditions allowing for endoproteolytic cleavage after Xaa and formation of an endoprotease target polypeptide-acyl-intermediate, adding an appropriate nucleophile, and upon nucleophilic attack and binding of said nucleophile to the C-terminus of the target polypeptide releasing the mutated trypsin from the endoprotease target polypeptide-acyl intermediate.

Preferably the above-described mutant trypsins and the above-described polypeptides comprising TYR-ARG-Xaa-HIS, wherein Xaa is any amino acid, are used in a method of C-terminal polypeptide modification by transacylation, e.g. in a method for peptide ligation. In a preferred embodiment the present invention therefore relates to a method of producing a C-terminally transacylated target polypeptide comprising the steps of: (a) providing a polypeptide comprising a target polypeptide and a restriction site peptide comprising the recognition site TYR-ARG-Xaa-HIS, wherein Xaa is any amino acid, (b) bringing said peptide into contact with a trypsin mutant according to the present invention under conditions allowing for endoproteolytic cleavage after TYR and formation of an endoprotease target polypeptide-acyl-intermediate, (c) adding an appropriate nucleophile and (d) upon nucleophilic attack and binding of said nucleophile to the C-terminus of the target polypeptide releasing the mutated trypsin from the endoprotease target polypeptide-acyl intermediate.

As used herein, a nucleophile is a molecule that donates a pair of electrons to an electron acceptor, in this case the carboxyl carbon of the peptide-acyl-enzyme intermediate, to form a covalent bond.

Preferably the nucleophile is selected from the group consisting of primary amines, imines, secondary amines, thiol and hydroxyl. Suitable nucleophiles for example include amino acids; amino acid derivatives, such as amino acid esters and amino acid amides), amines, such as ammonia, or benzyl amines.

The terms "transacylation" or "transacylated" are used to indicate that the C-terminal amino acid of the target polypeptide (TYR) is bound via covalent bond to the nucleophile. Where the nucleophile is a thiol the transacylation is a thiolation, where the nucleophile comprises a hydroxylic group the transacylation is an esterification and where the nucleophile is an amine the transacylation results in a transamidation. Transamidation reactions are very important and represent a preferred embodiment according to the present invention.

As the skilled artisan will readily appreciate, appropriate nucleophiles may furthermore comprise modifications which introduce desired properties to the C-terminal end of an appropriate target polypeptide. Preferably the present invention relates to a nucleophile comprising a modification that is selected from the group consisting of a peptide, a peptide amide, a label, a labeled amino acid amide, a labeled peptide, a labeled peptide amide, a non-natural amino acid, and polyethyleneglycole.

The term label is well-known to the skilled artisan and can be any desired structure of interest. Preferably such label can be selected from any known detectable groups, I such as dyes, luminescent labeling groups such as chemiluminescent groups e.g. acridinium esters or dioxetanes, or fluorescent dyes e.g. fluorescein, coumarin, rhodamine, oxazine, resorufin, cyanine and derivatives thereof. Other examples of labeling groups are luminescent metal complexes such as ruthenium or europium complexes, enzymes as used for CEDIA (Cloned Enzyme Donor Immunoassay, e. g. EP-A-0061888), and radioisotopes/chelators

Another preferred group of labels of interest for example comprises one partner of a bioaffine binding pair. While performing an assay this kind of label interacts I specifically and preferably non-covalent with the other partner of the bioaffine binding pair. Examples of suitable binding partners of bioaffine binding pairs are hapten or antigen/antibody, biotin or biotin analogues such as aminobiotin, iminobiotin or destheiobiotin/avidin or streptavidin, sugar/lectin, nucleic acid or nucleic acid analogue/complementary nucleic acid, receptor/ligand e.g. steroid hormone receptor/steroid hormone. Preferred labels within this group are selected from hapten, antigen and hormone. Especially preferred labels are haptens like digoxin and biotin and analogues thereof.

Another group of preferred modifications are non-natural amino acids and derivatives thereof. Most interesting are non-natural amino acids containing functional groups, which are orthogonal to the natural amino acids, e.g. aldehyde functions, hydrazines, hydrazides, azids, and α-halogen-ketones.

In case the nucleophile comprises polyethyleneglycole (PEG), this PEG preferably has a molecular weight in the range of 2.000 Da to 50.000 Da. The PEG may be linear or branched. More preferred the PEG will be in the molecular weight range from 10,000 Da to 40,000 Da. Preferably the nucleophilic group of such nucleophile comprising PEG will be an arginine or a lysine having a free N-terminal α-amino group. This arginine or this lysine also may be the N-terminus of a peptide. Preferably such pegylated nucleophile is selected from the group consisting of Arg-Xaa-His-PEG, Arg-Xaa-His-Ala-PEG, Lys-Xaa-His-PEG, Lys-Xaa-His-Ala-PEG and Arg-Xaa-His-Xaa-PEG, wherein Xaa may be any natural or non-natural α-amino carboxylic acid. The PEG-modified α-amino carboxylic acid may comprise one or two PEG molecule(s) bound to one or to both of these amino groups, respectively. The skilled artisan may select or design other appropriate PEG-modified nucleophiles, like a pegylated cysteine and others.

According to procedures known in the state of the art or according to the procedures given in the examples section and armed with the teaching of the present invention, it is now possible to obtain polynucleotide sequences coding for the trypsin mutants of the invention. Preferably the mutated trypsin according to the present invention is expressed as an inactive precursor (Zymogen) which is enzymatically cleaved to result in the active enzyme. **In** a further embodiment the present invention relates to a nucleotide sequence coding for the mutated trypsin comprising an amino acid substitution at position both at K60 and D189, and amino acid substitution at position Y39 and Y59.

The present invention further includes an expression vector comprising a nucleic acid sequence according to the present invention operably linked to a promoter sequence capable of directing its expression in a host cell.

The present invention further includes an expression vector comprising a nucleic acid sequence according to the present invention operably linked to a promoter sequence capable of directing its expression in a host cell. Preferred vectors are plasmids such as pST and pYT shown in Figure 1.

Expression vectors useful in the present invention typically contain an origin of replication, a promoter located upstream in the DNA sequence, and are followed by the DNA sequence coding for a trypsin mutant, followed by transcription termination sequences and the remaining vector. The expression vectors may also include other DNA sequences known in the art, for example, stability leader sequences which provide for stability of the expression product, secretory leader sequences which provide for secretion of the expression product, sequences which allow expression of the structural gene to be modulated (e.g., by the presence or absence of nutrients or other inducers in the growth medium), marking sequences which are capable of providing phenotypic selection in transformed host cells, and the sequences which provide sites for cleavage by restriction endonucleases.

The characteristics of the actual expression vector used must be compatible with the host cell, which is to be employed. For example, when cloning in an E.coli cell system, the expression vector should contain promoters isolated from the genome of E.coli cells (e.g., lac, or trp). Suitable origins of replication in E.coli various hosts include, for example, a ColE1 plasmid replication origin. Suitable promoters include, for example, lac and trp. It is also preferred that the expression vector includes a sequence coding for a selectable marker. The selectable marker is preferably an antibiotic resistance gene. As selectable markers, ampicillin resistance, or canamycin resistance may be conveniently employed. All of these materials are known in the art and are commercially available.

Suitable expression vectors containing the desired coding and control sequences may be constructed using standard recombinant DNA techniques known in the art, I many of which are described in Sambrook, J. et al., Molecular Cloning: A Laboratory Manual (1989).

The present invention additionally concerns host cells containing an expression vector which comprises a DNA sequence coding for the mutant trypsin according to the present invention. Preferred are the host cells containing an expression vector comprising one or more regulatory DNA sequences capable of directing the replication and/or the expression of, and operatively linked to a DNA sequence coding for, all or a functional part of mutant trypsin. Suitable host cells include, for example, E. coli HB101 (ATCC 33694) available from Promega (2800 Woods Hollow Road, Madison, WI, USA), XL1-Blue MRF available from Stratagene (11011 North Torrey Pine Road, La Jolla, CA, USA) and the like.

Expression vectors may be introduced into host cells by various methods known in the art. For example, transformation of host cells with expression vectors can be carried out by polyethylene glycol mediated protoplast transformation method (Sambrook et al. 1989). However, other methods for introducing expression vectors into host cells, for example, electroporation, holistic injection, or protoplast fusion, can also be employed.

Once an expression vector containing trypsin mutant has been introduced into an appropriate host cell, the host cell may be cultured under conditions permitting expression of the desired trypsin mutants. Host cells containing an expression vector which contains a DNA sequence coding for the trypsin mutant are, e.g., identified by one or more of the following general approaches: DNA hybridization, the presence or absence of marker gene functions, assessment of the level of transcription as measured by the production of trypsin mRNA transcripts in the host cell, and detection of the gene product immunologically.

It should, of course, be understood that not all expression vectors and DNA regulatory sequences would function equally well to express the DNA sequences of the present invention. Neither will all host cells function equally well with the same expression system. However, one of ordinary skill in the art will make a selection among expression vectors, DNA regulatory sequences, and host cells using the guidance provided herein without undue experimentation.

### Method for preparing N-terminally transacylated target polypeptide

Also provided is a method of preparing a N-terminal transacylated target polypeptide comprising the steps of: (a) providing a polypeptide comprising a target polypeptide and a tripeptide ARG-Xaa-HIS N-terminally fused to the target polypeptide, wherein Xaa is any amino acid, (b) providing a reactive acyl donor substrate, (c) bringing the reactive acyl donor substrate into contact with the mutated trypsin of the present invention under conditions allowing for acylation of Ser195 of the mutated trypsin by the reactive acyl donor substrate, (d) adding the polypeptide from step (a), (e) upon nucleophilic attack and binding of said polypeptide, releasing the mutated trypsin and forming a N-terminally transacylated target polypeptide.

A polypeptide is provided comprising a target polypeptide. To this target polypeptide a tripeptide ARG-Xaa-HIS is N-terminally fused, wherein Xaa is any amino acid.

Further a reactive acyl donor substrate is provided. The reactive acyl donor substrate is not further restricted as long as it is able to acylate Ser195 of the mutated trypsin according to present invention. Examples of reactive acyl donor substrates are Ac-Tyr-OMe, Bz-Xaa-OGp, Bz-Xaa-OPh, Bz-Xaa-OBzl or correspondig peptide esters; e.g. peptidyl-OGp, peptidyl-OBzl or peptidyl-tyrosyl (methyl) esters.

Preferably the acyl donor substrate is an OGp ester (OGp: 4-guanidinophenyl ester). Preferred examples are Boc-Ala-OGp, 4-pentynoic acid-OGp, Bz-Gly-OGp, Norb-Ahx-OGp or Dan-Ahx-OGp.

In the next step the reactive acyl donor substrate is brought into contact with the mutated trypsin of the present invention under conditions allowing for acylation of Ser195 of the mutated trypsin by the reactive acyl donor substrate.

To the acylated mutated trypsin then the polypeptide comprising a target polypeptide and a tripeptide ARG-Xaa-HIS N-terminally fused to the target polypeptide is added. Upon nucleophilic attack and binding of said polypeptide, the mutated trypsin is released and a N-terminally transacylated target polypeptide is formed.

The following examples do not restrict the scope of the invention and only help to understand and explain the features of the present invention. Deviations and modifications on these particular examples, particular with regard to other aspects of the invention, can be made without departing from the scope of the invention.

### Examples

### Vectors / Plasmids

The Escherichia coli vector pST was used for site-directed mutagenesis of trypsin. This vector is equipped with ampicillin resistance and contains the sequence for anionic rat tripsinogen II, which is a fusion of a "α-factor-leader" and a GAPDH/ADH promoter. This construct is flanked by an interface for the restriction enzymes Bam HI and Sal I. The trypsin variants were expressed in the Saccharomyces cerevisiae expression vector pYT and selected for leucine and uracil. Both plasmids, pST and pYT, contain the ampicillin resistance gene as a selection marker (see Figure 1).

### Culture media

Different culture media were used to cultivate S. cerevisiae E. coli (Table 1). The trypsin variants were expressed in a minimal medium. Leucine and uracil were not added.

**Table 1: Media compositions YPD (yeast extract peptone dextrose), SC (synthetic complete), and LB.**

| | **SC** - **ura - leu** | **[g/l]** |
|---|---|---|
| | Yeast Nitrogen base | **6,6** |
| | 0,37 g Adenine, 1 g His*HCl, 1 g Arg*HCl, 1 g Met, 1,5 g Tyr, 1,5 g Ile, 1,5 g Lys*HCl, 2,5 g Phe, 5 g Asp, 7,5 g Val, 10 g Thr, 18,75 g Ser | **1,3** |
| **- leu** | Leucine | **0,036** |
| **- ura** | Uracil | **0,024** |
| | Glucose (Roth) | **80** |
| | Agar (AppliChem, Darmstadt) | **20** |

| | **LB** | **[g/l]** |
|---|---|---|
| | Bacto pepton (GIBCOBRL^{®}, Scotland) | **10** |
| | NaCl | **10** |
| | Yeast extract (SERVA, Heidelberg) | **5** |
| | Agar (AppliChem, Darmstadt) | **20** |

| | **YPD** | **[g/l]** |
|---|---|---|
| | Yeast extract (SERVA, Heidelberg) | **5** |
| | Bacto-Pepton (GIBCOBRL^{®}, Scotland) | **10** |

### Expression and purification of trypsin variants

The plasmid was transformed into S. cerevisiae by common methods. The yeasts were first crossed out on Sc-ura plates (four days, 30 °C). Some of the grown colonies were then transferred to Sc-leu plates and cultivated at 30 °C for two days.

The yeast colonies grown on the Sc-leu plates were then incubated in Sc-leu liquid medium for another three days at 30 °C before being inoculated into the main culture.

The main culture was in non-selective YPD medium. The expression of the variant trypsinogen was induced by lowering the glucose content from 8% in the Sc-leu medium to 1.2% in the YPD medium.

Since the trypsinogen is released into the medium due to the α-factor leader sequence in the pYT vector, the cellular components were centrifuged (SORVALL^{®} RC 5B PLUS, 10 min at 1704xg) and the supernatant processed.

The pH was adjusted to pH 4 with a 1 M HCl solution. The precipitated polypeptides and undissolved remaining cell debris were removed by centrifugation (SORVALL^{®} RC 5B PLUS, 15 min at 15337xg).

A cation exchange column equilibrated with 2 mM Na-acetate/100 mM acetic acid (Toyopearl 650 M (SUPELCO), pH 4.5) was loaded with the polypeptide solution. The elution was performed with 0.2 M Tris-HCl buffer at pH 8, the trypsinogen containing fractions were identified by SDS-polyacrylamide gel electrophoresis (SDS-PAGE), combined and narrowed to a volume of about 20 ml with Centriprep-10 concentrators (AMICON). The trypsinogen solution was adjusted to pH 6 with 1 M NaOH and activated with 0.4 U enterokinase (EK 4, New England Biolabs). Activation was tracked via SDS-PAGE and was completed after three to four days.

After activation of the trypsinogen, the remaining trypsinogen, propeptide and activated trypsin were separated by perfusion chromatography (BioCad 700) using an anion exchange column. Eluted with a 1.5 M NaCl gradient of 0-30%. The collected trypsin fractions were dialyzed against 1 mM HCl at 4°C, concentrated and analyzed using SDS-PAGE (Figure 2).

### Synthesis of OGp-ester

0.01 mmol carboxylic acid was dissolved with 0.01 mmol isobutyl chloroformate at -20 °C in DMF and mixed with 0.03 mmol triethylamine. After 20 min 0.02 mmol HOGp(Boc,Boc)*Tosyl was added. The reaction time was one hour. The product was processed by adding ethyl acetate and shaking with a 5% KHSO₄ solution (m/v). Purification was performed using preparative HPLC using a water-acetonitrile gradient at 220 nm and a flow rate of 9 ml/min. A reverse phase column was used (C8, Merck). The Boc protection groups were split off with 50% TFA (v/v) in DCM. The TFA-DCM mixture was then evaporated. The product was precipitated in ether.

### Peptide synthesis

The peptides were synthesized according to the orthogonal Fmoc/t-butyl strategy. The resin used was 2-chlorotrityl resin. The peptide syntheses were carried out in polypropylene reactors. The carboxy component was activated with TBTU and DIPEA in DMF. Fmoc was separated with 20% (v/v) piperidine in DMF.

In the case of the peptides modified on the lysine side chain, Fmoc-Lys(Dde) was used. The cleavage of the Dde side chain protection group was carried out by means of 2% hydrazine in DMF or hydroxylamine on the resin and subsequent coupling of carboxyfluorescein. Benzoylations of the N-termini of the corresponding peptides were carried out with TBTU-activated benzoic acid directly on the resin after splitting off the Fmoc protective group.

The representation of all other modified peptides is described below.

The peptides were split off from the resin with 95% TFA (v/v), 2.5% TIS (v/v), 2.5% water (v/v). After evaporation of the TFA, the peptide was precipitated with ether. The purification was carried out using preparative HPLC in an acetonitrile-water gradient. Acetonitrile and water each contained 0.1% TFA (v/v).

### Specificity tests - hydrolysis

The hydrolysis kinetic assays were performed with the trypsin variant K60E/D189K/Y59H/Y39H. The peptides Bz-AAYRAAG-OH, Bz-AAYRHAAG-OH, Bz-AAYRAHAAG-OH were hydrolysed in the presence of zinc or EDTA. The reaction mixture consisted of 1 mM peptide, 10 µM trypsin variant, 0.1 M HEPES, 0.1 M NaCl, 0.01 M CaCl₂, 100 µM ZnCl₂/EDTA; pH 8. Hydrolyses of the model peptides were analyzed using UPLC. For this purpose, aliquots were taken from the reaction mixtures after certain time intervals and mixed with 50% (v/v) acetic acid. The time intervals were adapted to the conversion rates of the respective trypsin variants to be tested, so that the hydrolysis rates could be determined in the linear range. The data recorded are the results of at least two independent measurement series. Under no circumstances were the results more than 5% different (Fig 3.).

As shown in Figure 3, the trypsin variant Y39H/Y59H/K60E/D189K is capable of hydrolyzing the selected peptides with histidine side chains in positions P₂' and P₃'. The comparison peptide without histidine is also converted in the sequence (Bz-AAYRAAG-OH). In principle, differences in the hydrolysis rate and the zinc-mediated inducibility of the hydrolysis reaction depending on the position of the histidine side chain (P₂' or P₃') can be recognized. The trypsin variant Y39H/Y59H/K60E/D189K converts the peptide Bz-AAYRAHAAG-OH with the highest initial hydrolysis rate; a high conversion rate is achieved exclusively in the presence of zinc ions.

### C-terminal modification

### Specificity tests - aminolysis

Kinetic specificity tests were performed using the transamidation reaction (aminolysis). For this purpose acyl donor/acyl acceptor pairs were used, which each had the same recognition sequence.

An example of an acyl donor/acceptor pair would be: Bz-AAYRAHAG/RAHAK(DNP). Positions P₁, P₁', P₂', P₃' (Fig. 4) were varied.

On the basis of these investigations, the recognition sequence is Bz-PGGYRAHAG. Instead of alanine in position P₂', other amino acids can also be used (lysine, arginine). Instead of tyrosine in position P₁, the amino acids leucine, phenylalanine, alanine, tryptophan can also be used.

### Model peptide

Transamidations were performed with 0.1 mM acyl donor and 4 mM acyl acceptor (nucleophile). The trypsin variant was used in a concentration of 10-20 µM. As buffer 0.1 M HEPES, 0.1 M NaCl, 0.01 M CaCl₂, 100 µM ZnCl₂/EDTA; pH 7.8 were used.

### Modification of model peptide

When using the nucleophile RAHAK(5,6CF) in excess (4 mM; Figure 5) the product yields of Bz-AAYRAHAK(5,6CF) were 27%.

### C-terminal modification Fab fragment

Transamidations were performed with 0.1 mM acyl donor (anti-Her2-Fab-YRAH; Trastuzumab related Fab) and 2 mM RAHAK(DNP) (nucleophile). The anti-Her2-Fab-YRAH comprises a heavy chain variable region VH comprising an amino acid sequence of SEQ ID NO: 2 and a light chain variable region VL comprising an amino acid sequence of SEQ ID NO:3.The trypsin variant was used in a concentration of 5 µM. As buffer 0.1 M HEPES, 0.1 M NaCl, 0.01 M CaCl₂, 100 µM ZnCl₂/EDTA; pH 7.8 were used (cf. Fig. 6).

### N-terminal modification (acyl transfer)

### The required OGp esters were produced as follows:

0.01 mmol carboxylic acid was dissolved with 0.01 mmol isobutyl chloroformate at -20 °C in DMF and mixed with 0.03 mmol triethylamine. After 20 min 0.02 mmol HOGp(Boc,Boc)*Tosyl was added. The reaction time was one hour. The product was processed by adding ethyl acetate and shaking with a 5% KHSO₄ solution (m/v). Purification was performed using preparative HPLC using a water-acetonitrile gradient at 220 nm and a flow rate of 9 ml/min. A reverse phase column was used (C8, Merck). The Boc protection groups were split off with 50% TFA (v/v) in DCM. The TFA-DCM mixture was then evaporated. The product was precipitated in ether.

### N-terminal modification

Acyl transfer reactions were performed with Boc-Ala-OGp and 4-pentynoic acid-OGp as acyl donors. AHAAG, AAHAG, RHAK(5,6CF), RAHAK(5,6CF), RAAHK(5,6CF) and RHAAAK(5,6CF) were used as acyl acceptors. The final concentrations were 0.1-4 mM ester, 0.1-4 mM acyl acceptor (nucleophile) and 10-20 µM enzyme variant. As buffer 0.1 M HEPES, 0.1 M NaCl, 0.01 M CaCl₂, 100 µM ZnCl₂/EDTA; pH 7.

The substrate ester Boc-Ala-OGp was used as acyl donor. The maximum time required to completely hydrolyze the ester substrate Boc-Ala-OGp enzymatically in the absence of the nucleophile was determined in advance. Depending on the concentrations of acyl donor and acyl acceptor and taking into account the determined reaction time of 80 minutes, it was determined which sequence with the highest efficiency is converted to Boc-A-RXXXK(5,6CF) (Figure 7).

Figure 7 shows a clear preference of the trypsin variant Y39H/Y59H/K60E/D189K for histidine residues in the P₃' position. Yields in a range of 54% -100% are achieved with the acyl acceptor RAHAK(5,6CF). The synthesis efficiency or yield is significantly dependent on the ratio of acyl donor and acyl acceptor used under the selected conditions; with a 5-fold excess of acyl acceptor, the acyl transfer reaction is quantitative. The ligation reactions of Boc-Ala-OGp and acyl acceptor peptides with histidine side chains in positions P₂' and P₄' show in principle the same dependence of the product yield on the nucleophile concentration. However, as expected from the initial hydrolysis reactions and specificity studies, the use of acyl acceptors with histidine side chains in positions P₂' and P₄' resulted in significantly lower product yields of 16%-38% for RAAHK(5.6CF) and only 6%-17% for RHAAK(5.6CF).

### Modification of cyclophilin18

For the N-terminal derivatization of cyclophilin18 the substrate polypeptide Strep-YRAH-cyclophilin18-YRHAAG-OH, 4-pentynoic acid-OGp was used as acyl donor. The cyclophilin18 carried the N-terminal recognition sequence -YRAH- for trypsin variant Y39H/Y59H/K60E/D189K catalyzable modification reactions. A successful modification at the N-terminal end could be demonstrated (Fig. 8). Strep-YRAH-cyclophilin18 has an amino acid sequence of SEQ ID NO: 4

As already shown in polyacrylamide gel electrophoresis (Figure 9A), non-proteolyzed Strep-YRAH-Cyp18-YRHAAG-OH (20,175 Da; Figure 9B) was present after a reaction time of two hours. The difference between the complete construct and the cleavage product RAH-Cyp18-YRHAAG-OH corresponds to the sequence H-GWSHPQFEKY-OH, i.e. the strep fusion peptide; no hydrolysis of the YRH sequence additionally present in the polypeptide took place.

Figure 8C shows the result of the mass spectrometric analysis of the acyl transfer between 4-pentynoic acid OGp and Strep-YRAH-Cyp18-YRHAAG-OH reaction after one hour. Here it can be seen that both the starting material Strep-YRAH-Cyp18-YRH (20,175 Da), the intermediate RAH-Cyp18-YRH (18,915 Da) and the acyl transfer product PS-RAH-Cyp18-YRH (18,995 Da) are present in the reaction batch; the reaction is therefore not yet finished after 60 min.

### Modification of RAH-Trastuzumab

For the N-terminal derivatization of the full length anti-Her2-antibody RAH-Trastuzumab, Dan-Ahx-OGp was used as acyl donor. The anti-Her2-antibody RAH-Trastuzumab comprises a heavy chain variable region VH comprising an amino acid sequence of SEQ **ID** NO:5 and a light chain variable region VL comprising an amino acid sequence of SEQ **ID** NO:6. The antibody carried the enzyme specific N-terminal recognition sequence -RAH- (this time without any further (purification) tag at the very N-terminus of Trastuzumab) for trypsin variant Y39H/Y59H/K60E/D189K catalyzable modification reactions. A successful modification at the N-terminal end could be demonstrated. Fig. 10 shows the kinetic of the derivatization reaction resulting in a quantitative yield of selectively N-terminal modified RAH-Trasuzumab; i.d. Dan-Ahx-RAH-Trastuzumab with a DAR 2. DAR 0, DAR 1 and DAR 2 define the Drug-Antibody-Ratio. Accordingly, quantitative yield of DAR 2 Dan-Ahx-RAH-Trastuzumab corresponds to full derivatization of the two N-termini of RAH-Trastuzumab via trypsin variant catalysis.

### The following sequences are disclosed herein:

Rat anionic trypsin II (SEQ ID NO: 1): 
IVGGYTCQEN SVPYQVSLNS GYHFCGGSLI NDQWVVSAAH CYKSRIQVRL
GEHNINVLEG NEQFVNAAKI IKHPNFDRKT LNNDIMLIKL SSPVKLNARV ATVALPSSCA
PAGTQCLISG WGNTLSSGVN EPDLLQCLDA PLLPQADCEA SYPGKITDNM
VCVGFLEGGK DSCQGDSGGP VVCNGELQGI VSWGYGCALP DNPGVYTKVC
NYVDWIQDTI AAN
anti-Her2-Fab-YRAH, Heavy chain (SEQ ID NO: 2): 
EVKLQESGGG LVQPGGSLRL SCAASGFNIK DTYIHWVRQA PGKGLEWVAR
IYPTNGYTRY ADSVKGRFTI SADTSKNTAY LQMNSLRAED TAVYYCSRWG
GDGFYAMDYW GQGTLVTVSS ASTKGPSVFP LAPSSKSTSG GTAALGCLVK
DYFPEPVTVS WNSGALTSGV HTFPAVLQSS GLYSLSSVVT VPSSSLGTQT YICNVNHKPS
NTKVDKKVEP KSCADKPGG YRAHAGEQKL ISEEDL
anti-Her2-Fab-YRAH, Light chain (SEQ ID NO: 3): 
DIELTQSPSS LSASVGDRVT ITCRASQDVN TAVAWYQQKP GKAPKLLIYS ASFLYSGVPS
RFSGSRSGTD FTLTISSLQP EDFATYYCQQ HYTTPPTFGQ GTKLEIKRTV AAPSVFIFPP
SDEQLKSGTA SVVCLLNNFY PREAKVQWKV DNALQSGNSQ ESVTEQDSKD
STYSLSSTLT LSKADYEKHK VYACEVTHQG LSSPVTKSFN RGEC
Strep-YRAH-cyclophilin18 (SEQ ID NO: 4): 
GWSHPQFEKY RAHAVNPTVF FDIAVDGEPL GRVSFELFAD KVPKTAENFR
ALSTGEKGFG YKGSCFHRII PGFMCQGGDF TRHNGTGGKS IYGEKFEDEN FILKHTGPGI
LSMANAGPNT NGSQFFICTA KTEWLDGKHV VFGKVKEGMN IVEAMERFGS
RNGKTSKKIT IADCGQLAYR HAAG
anti-Her2-antibody RAH-Trastuzumab, Heavy chain (SEQ ID NO: 5): 
EVQLVESGGG LVQPGGSLRL SCAASGFNIK DTYIHWVRQA PGKGLEWVAR
IYPTNGYTRY ADSVKGRFTI SADTSKNTAY LQMNSLRAED TAVYYCSRWG
GDGFYAMDYW GQGTLVTVSS ASTKGPSVFP LAPSSKSTSG GTAALGCLVK
DYFPEPVTVS WNSGALTSGV HTFPAVLQSS GLYSLSSVVT VPSSSLGTQT YICNVNHKPS
NTKVDKKVEP KSCDKTHTCP PCPAPELLGG
PSVFLFPPKP KDTLMISRTP EVTCVVVDVS HEDPEVKFNW YVDGVEVHNA
KTKPREEQYN STYRVVSVLT VLHQDWLNGK EYKCKVSNKA LPAPIEKTIS
KAKGQPREPQ VYTLPPSREE MTKNQVSLTC LVKGFYPSDI AVEWESNGQP
ENNYKTTPPV LDSDGSFFLY SKLTVDKSRW QQGNVFSCSV MHEALHNHYT
QKSLSLSPG
anti-Her2-antibody RAH-Trastuzumab, Light chain (SEQ ID NO: 6):

RAHADIQMTQ SPSSLSASVG DRVTITCRAS QDVNTAVAWY QQKPGKAPKL LIYSASFLYS
GVPSRFSGSR SGTDFTLTIS SLQPEDFATY YCQQHYTTPP TFGQGTKVEI KRTVAAPSVF
IFPPSDEQLK SGTASVVCLL NNFYPREAKV QWKVDNALQS GNSQESVTEQ
DSKDSTYSLS STLTLSKADY EKHKVYACEV THQGLSSPVT KSFNRGEC

Further the following polypeptides are disclosed herein with the indicated modifications at the specified positions::

| | |
|---|---|
| Bz-YRAAG-OH | (SEQ ID NO: 7) |
| Bz-YRHAAG-OH | (SEQ ID NO: 8) |
| Bz-YRAHAAG-OH | (SEQ ID NO: 9) |
| Bz-AAYRAAHAG-OH | (SEQ ID NO: 10) |
| Bz-AAYRAHAAG-OH | (SEQ ID NO: 11) |
| Bz-AAYRHAAAG-OH | (SEQ ID NO: 12) |
| AHAG | (SEQ ID NO: 13) |
| AAHAG | (SEQ ID NO: 14) |
| RAHAK(CF) | (SEQ ID NO: 15) |
| RAAHK(CF) | (SEQ ID NO: 16) |
| RHAK(CF) | (SEQ ID NO: 17) |
| RAHAK(DNP) | (SEQ ID NO: 18) |
| BzPGGYRKDAG | (SEQ ID NO: 19) |
| BzPGGYRKKAG | (SEQ ID NO: 20) |
| BzPGGYRKNAG | (SEQ ID NO: 21) |
| BzPGGYRKAAG | (SEQ ID NO: 22) |
| BzPGGYRRHAG | (SEQ ID NO: 23) |
| BzPGGYDKHAG | (SEQ ID NO: 24) |
| BzPGGYAKHAG | (SEQ ID NO: 25) |
| BzPGGARKHAG | (SEQ ID NO: 26) |
| BzPGGDRKHAG | (SEQ ID NO: 27) |
| BzPGGRRKHAG | (SEQ ID NO: 28) |
| BzPGGLRKHAG | (SEQ ID NO: 29) |
| BzPGGWRKHAG | (SEQ ID NO: 30) |
| BzPGGFRKHAG | (SEQ ID NO: 31) |
| BzPGGYRKHAG | (SEQ ID NO: 32) |
| BzPGGYRADAG | (SEQ ID NO: 33) |
| BzPGGYRAKAG | (SEQ ID NO: 34) |
| BzPGGYRANAG | (SEQ ID NO: 35) |
| BzPGGYRAAAG | (SEQ ID NO: 36) |
| BzPGGYKAHAG | (SEQ ID NO: 37) |
| BzPGGYDAHAG | (SEQ ID NO: 38) |
| BzPGGYAAHAG | (SEQ ID NO: 39) |
| BzPGGARAHAG | (SEQ ID NO: 40) |
| BzPGGDRAHAG | (SEQ ID NO: 41) |
| BzPGGRRAHAG | (SEQ ID NO: 42) |
| BzPGGWRAHAG | (SEQ ID NO: 43) |
| BzPGGFRAHAG | (SEQ ID NO: 44) |
| BzPGGYRAHAG | (SEQ ID NO: 45) |
| RKDAK(DNP) | (SEQ ID NO: 46) |
| RKKAK(DNP) | (SEQ ID NO: 47) |
| BRKNAK(DNP) | (SEQ ID NO: 48) |
| RKAAK(DNP) | (SEQ ID NO: 49) |
| RRHAK(DNP) | (SEQ ID NO: 50) |
| DKHAK(DNP) | (SEQ ID NO: 51) |
| AKHAK(DNP) | (SEQ ID NO: 52) |
| RKHAK(DNP) | (SEQ ID NO: 53) |
| RADAK(DNP) | (SEQ ID NO: 54) |
| RAKAK(DNP) | (SEQ ID NO: 55) |
| RANAK(DNP) | (SEQ ID NO: 56) |
| RAAAK(DNP) | (SEQ ID NO: 57) |
| KAHAK(DNP) | (SEQ ID NO: 58) |
| DAHAK(DNP) | (SEQ ID NO: 59) |
| AAHAK(DNP) | (SEQ ID NO: 60) |

Wherein the modifications have the following meaning:
- Bz-: benzoyl
- Boc-: tert-butyloxycarbonyl
- OGp-: O-guanidinophenyl
- DNP-: dinitrophenyl
- CF-: carboxyfluorescein

## Claims

1. A mutated trypsin comprising an amino acid substitution both at position K60 and D189, and amino acid substitution at position Y39 and Y59, wherein positions K60, D189, Y39, and Y59 according to the chymotrypsin nomenclature correspond to positions 43, 171, 22, and 42, respectively, of the trypsin sequence given in SEQ **ID** NO:1.

2. The mutated trypsin of claim 1, wherein position K60 is substituted by E or D.

3. The mutated trypsin of any of claims 1 to 2, wherein position D189 is substituted by K, H or R.

4. The mutated trypsin of any of claims 1 to 3, wherein position Y39 is substituted by K, H or R.

5. The mutated trypsin of any of claims 1 to 4, wherein position Y59 is substituted by K, H or R.

6. Use of a polypeptide comprising a target polypeptide and a restriction site peptide comprising the recognition site TYR-ARG-Xaa-HIS, wherein Xaa is any amino acid, wherein said restriction site peptide overlaps with the target polypeptide by the amino acid TYR at the C-terminal end of said target polypeptide as a substrate of a mutated trypsin according to any of claims 1 to 5.

7. A nucleotide sequence coding for the mutated trypsin as defined in any of claims 1 to 5.

8. A method for preparing a C-terminal transacylated target polypeptide comprising the steps of:
(a) providing a polypeptide comprising a target polypeptide and a restriction site peptide comprising the recognition site TYR-ARG-Xaa-HIS, wherein Xaa is any amino acid, and wherein said restriction site peptide overlaps with the target polypeptide by the amino acid TYR at the C-terminal end of said target polypeptide,
(b) bringing said peptide into contact with a trypsin mutant according to any of claims 1 to 5 under conditions allowing for endoproteolytic cleavage after TYR and formation of an endoprotease target polypeptide peptide-acyl-intermediate,
(c) adding an appropriate nucleophile and
(d) upon nucleophilic attack and binding of said nucleophile to the C- terminus of the target polypeptide releasing the mutated trypsin from the endoprotease target polypeptide-acyl-intermediate.

9. The method of claim 8, wherein said nucleophile is selected from the group consisting of primary amines, imines, secondary amines, thiol and hydroxyl.

10. The method of claim 9, wherein said nucleophile comprising modification is selected from the group consisting of an amino acid amide, a peptide, a peptide amide, a label, a labeled amino acid amide, a labeled peptide, a labeled peptide amide, and polyethyleneglycole.

11. The method of any of claims 8 to 10, wherein the nucleophile is a click-anchor comprising at least one additional functional group A, wherein the at least functional group A is capable under appropriate conditions to form at least one covalent bond to a complementary functional group B.

12. The method of claim 11, wherein the at least one additional functional group A of the click-anchor is selected from the group consisting of an aldehyde, an azide, a C-C double bond, a C-C triple bond, a C-N triple bond, a diene, an imine, a ketone, a tetrazine, a thioketone and a thiole.

13. The method of claim 11 or 12, wherein the functional group B is selected from the group consisting of an aminooxy, an azide, a C-C double bond, a C-C triple bond, a O-Chinonmethid, a C-N triple bond, a cystamine, a diene, a hydrazide, an iodophenyl, a nitrilimine, a nitrilioxide, a phosphine, a phosphite, a tetrazine, a thiocarboxylic acid and a thiole.

14. A method for preparing a N-terminal transacylated target polypeptide comprising the steps of:(a) providing a polypeptide comprising a target polypeptide and a tripeptide ARG-Xaa-HIS N-terminally fused to the target polypeptide, wherein Xaa is any amino acid,
(b) providing a reactive acyl donor substrate,
(c) bringing the reactive acyl donor substrate into contact with the mutated trypsin according to any of claims 1 to 5 under conditions allowing for acylation of Ser195 of the mutated trypsin by the reactive acyl donor substrate,
(d) adding the polypeptide from step (a),
(e) upon nucleophilic attack and binding of said polypeptide, releasing the mutated trypsin and forming a N-terminally transacylated target polypeptide.

## Patentansprüche

1. Mutiertes Trypsin, umfassend eine Aminosäuresubstitution sowohl an Position K60 als auch an Position D189 und eine Aminosäuresubstitution an Position Y39 und Y59, wobei die Positionen K60, D189, Y39 und Y59 nach der Chymotrypsin-Nomenklatur den Positionen 43, 171, 22 bzw. 42 der in SEQ ID Nr. 1 angegebenen Trypsin-Sequenz entsprechen.

2. Mutiertes Trypsin nach Anspruch 1, wobei Position K60 durch E oder D substituiert ist.

3. Mutiertes Trypsin nach einem der Ansprüche 1 bis 2, wobei Position D189 durch K, H oder R substituiert ist.

4. Mutiertes Trypsin nach einem der Ansprüche 1 bis 3, wobei Position Y39 durch K, H oder R substituiert ist.

5. Mutiertes Trypsin nach einem der Ansprüche 1 bis 4, wobei Position Y59 durch K, H oder R substituiert ist.

6. Verwendung eines ein Zielpolypeptid und ein Restriktionsstellenpeptid umfassenden Polypeptids, das die Erkennungsstelle TYR-ARG-Xaa-HIS umfasst, wobei Xaa eine beliebige Aminosäure ist, wobei das Restriktionsstellenpeptid mit dem Zielpolypeptid mit der Aminosäure TYR am C-terminalen Ende des Zielpolypeptids überlappt, als Substrat eines mutierten Trypsins nach einem der Ansprüche 1 bis 5.

7. Nucleotidsequenz, die das mutierte Trypsin, wie definiert in einem der Ansprüche 1 bis 5, codiert.

8. Verfahren zur Herstellung eines C-terminal transacylierten Zielpolypeptids, umfassend die folgenden Schritte:
(a) Bereitstellen eines ein Zielpolypeptid und ein Restriktionsstellenpeptid umfassenden Polypeptids, das die Erkennungsstelle ARG-Xaa-HIS umfasst, wobei Xaa eine beliebige Aminosäure ist, und wobei das Restriktionsstellenpeptid mit dem Zielpolypeptid mit der Aminosäure TYR am C-terminalen Ende des Zielpolypeptids überlappt,
(b) Inkontaktbringen des Peptids mit einer Trypsin-Mutante nach einem der Ansprüche 1 bis 5 unter Bedingungen, die die endoproteolytische Spaltung nach TYR und die Bildung eines Endoprotease-Zielpolypeptid-Peptid-Acyl-Zwischenprodukts ermöglichen,
(c) Hinzufügen eines geeigneten Nucleophils und
(d) beim nucleophilen Angriff und Binden des Nucleophils an den C-Terminus des Zielpolypeptids Freisetzen des mutierten Trypsins aus dem Endoprotease-Zielpolypeptid-Acyl-Zwischenprodukt.

9. Verfahren nach Anspruch 8, wobei das Nucleophil ausgewählt ist aus der Gruppe bestehend aus primären Aminen, Iminen, sekundären Aminen, Thiol und Hydroxyl.

10. Verfahren nach Anspruch 9, wobei das eine Modifizierung umfassende Nucleophil ausgewählt ist aus der Gruppe bestehend aus einem Aminosäureamid, einem Peptid, einem Peptidamid, einer Markierung, einem markierten Aminosäureamid, einem markierten Peptid, einem markierten Peptidamid und Polyethylenglykol.

11. Verfahren nach einem der Ansprüche 8 bis 10, wobei das Nucleophil ein Klick-Anker ist, der mindestens eine zusätzliche funktionelle Gruppe A umfasst, wobei die mindestens funktionelle Gruppe A unter geeigneten Bedingungen imstande ist, mindestens eine kovalente Bindung zu einer komplementären funktionellen Gruppe B auszubilden.

12. Verfahren nach Anspruch 11, wobei die mindestens eine zusätzliche funktionelle Gruppe A des Klick-Ankers ausgewählt ist aus der Gruppe bestehend aus einem Aldehyd, einem Azid, einer C-C-Doppelbindung, einer C-C-Dreifachbindung, einer C-N-Dreifachbindung, einem Dien, einem Imin, einem Keton, einem Tetrazin, einem Thioketon und einem Thiol.

13. Verfahren nach Anspruch 11 oder 12, wobei die funktionelle Gruppe B ausgewählt ist aus der Gruppe bestehend aus einem Aminooxy, einem Azid, einer C-C-Doppelbindung, einer C-C-Dreifachbindung, einem O-Chinonmethid, einer C-N-Dreifachbindung, einem Cystamin, einem Dien, einem Hydrazid, einem lodphenyl, einem Nitrilimin, einem Nitriloxid, einem Phosphan, einem Phosphit, einem Tetrazin, einer Thiocarbonsäure und einem Thiol.

14. Verfahren zur Herstellung eines N-terminal transacylierten Zielpolypeptids, umfassend die folgenden Schritte: (a) Bereitstellen eines Polypeptids, umfassend ein Zielpolypeptid und ein N-terminal an das Zielpolypeptid kondensiertes Tripeptid Arg-Xaa-His, wobei Xaa eine beliebige Aminosäure ist,
(b) Bereitstellen eines reaktiven Acyldonator-Substrats,
(c) Inkontaktbringen des reaktiven Acyldonator-Substrats mit dem mutierten Trypsin nach einem der Ansprüche 1 bis 5 unter Bedingungen, die die Acylierung von Ser195 des mutierten Trypsins durch das reaktive Acyldonator-Substrat ermöglichen,
(d) Hinzufügen des Polypeptids aus Schritt (a),
(e) beim nucleophilen Angriff und Binden des Polypeptids Freisetzen des mutierten Trypsins und Bilden eines N-terminal transacylierten Zielpolypeptids.

## Revendications

1. Trypsine mutée comprenant une substitution d'acide aminé à la fois à la position K60 et D189, et une substitution d'acide aminé à la position Y39 et Y59, dans laquelle les positions K60, 0189, Y39 et Y59 selon la nomenclature de la chymotrypsine correspondent respectivement aux positions 43, 171, 22 et 42 de la séquence de trypsine donnée dans la SEQ ID NO:1.

2. Trypsine mutée selon la revendication 1, dans laquelle la position K60 est substituée par E ou D.

3. Trypsine mutée selon l'une quelconque des revendications 1 à 2, dans laquelle la position D189 est substituée par K, H ou R.

4. Trypsine mutée selon l'une quelconque des revendications 1 à 3, dans laquelle la position Y39 est substituée par K, H ou R.

5. Trypsine mutée selon l'une quelconque des revendications 1 à 4, dans laquelle la position Y59 est substituée par K, H ou R.

6. Utilisation d'un polypeptide comprenant un polypeptide cible et un peptide de site de restriction comprenant le site de reconnaissance TYR-ARG-Xaa-HIS, Xaa étant un acide aminé quelconque, ledit peptide de site de restriction chevauchant le polypeptide cible par l'acide aminé TYR à l'extrémité C-terminale dudit polypeptide cible, comme substrat d'une trypsine mutée selon l'une quelconque des revendications 1 à 5.

7. Séquence nucléotidique codant pour la trypsine mutée telle que définie dans l'une quelconque des revendications 1 à 5.

8. Procédé de préparation d'un polypeptide cible transacylé en C-terminal comprenant les étapes consistant à :
(a) fournir un polypeptide comprenant un polypeptide cible et un peptide de site de restriction comprenant le site de reconnaissance TYR-ARG-Xaa-HIS, Xaa étant un acide aminé quelconque, et ledit peptide de site de restriction chevauchant le polypeptide cible par l'acide aminé TYR à l'extrémité C-terminale dudit polypeptide cible,
(b) mettre ledit peptide en contact avec un mutant de trypsine selon l'une quelconque des revendications 1 à 5 dans des conditions permettant un clivage endoprotéolytique après TYR et la formation d'un intermédiaire acylé peptide-polypeptide cible d'endoprotéase,
(c) ajouter un nucléophile approprié, et
(d) lors de l'attaque nucléophile et de la liaison dudit nucléophile à l'extrémité C-terminale du polypeptide cible, libérer la trypsine mutée de l'intermédiaire acylé polypeptide cible d'endoprotéase.

9. Procédé selon la revendication 8, dans lequel ledit nucléophile est choisi dans le groupe constitué par les amines primaires, les imines, les amines secondaires, un thiol, et un hydroxyle.

10. Procédé selon la revendication 9, dans lequel ledit nucléophile comprenant une modification est choisi dans le groupe constitué par un amide d'acide aminé, un peptide, un amide de peptide, un marqueur, un amide d'acide aminé marqué, un peptide marqué, un amide de peptide marqué, et le polyéthylène glycol.

11. Procédé selon l'une quelconque des revendications 8 à 10, dans lequel le nucléophile est une ancre clic comprenant au moins un groupe fonctionnel supplémentaire A, l'au moins groupe fonctionnel A étant capable, dans des conditions appropriées, de former au moins une liaison covalente avec un groupe fonctionnel complémentaire B.

12. Procédé selon la revendication 11, dans lequel l'au moins un groupe fonctionnel supplémentaire A de l'ancre clic est choisi dans le groupe constitué par un aldéhyde, un azide, une double liaison C-C, une triple liaison C-C, une triple liaison C-N, un diène, une imine, une cétone, une tétrazine, une thiocétone, et un thiol.

13. Procédé selon la revendication 11 ou 12, dans lequel le groupe fonctionnel B est choisi dans le groupe constitué par un aminooxy, un azide, une double liaison C-C, une triple liaison C-C, un O-Quinone méthide, une triple liaison C-N, une cystamine, un diène, un hydrazide, un iodophényle, une nitrilimine, un oxyde de nitrile, une phosphine, un phosphite, une tétrazine, un acide thiocarboxylique, et un thiol.

14. Procédé de préparation d'un polypeptide cible transacylé en N-terminal comprenant les étapes consistant à : (a) fournir un polypeptide comprenant un polypeptide cible et un tripeptide ARG-Xaa-HIS fusionné en N-terminal au polypeptide cible, Xaa étant un acide aminé quelconque,
(b) fournir un substrat donneur d'acyle réactif,
(c) mettre le substrat donneur d'acyle réactif en contact avec la trypsine mutée selon l'une quelconque des revendications 1 à 5 dans des conditions permettant l'acylation de Ser195 de la trypsine mutée par le substrat donneur d'acyle réactif,
(d) ajouter le polypeptide de l'étape (a),
(e) lors de l'attaque nucléophile et de la liaison dudit polypeptide, libérer la trypsine mutée et former un polypeptide cible transacylé en N-terminal.
